# EUROPEAN PATENT APPLICATION

(11) **EP 4 018 930 A1**
(43) Date of publication of application: **29.06.2022**
(21) Application number: 21188953.0
(22) Date of filing: 30.07.2021
(51) Int. Cl.: A61B 5/262, A61B 5/00, A61B 5/268

(54) **NEURONAL DEVICE FOR OPTOGENETIC APPLICATIONS AND NEURONAL ACTIVITY MONITORING IN AN ANIMAL AND ITS USE**

(30) Priority: 28.12.2020 PT 2020116977
(71) Applicant: Universidade do Minho, 4704-553 Braga (PT)
(72) Inventor: GOMES CORREIA, José Higino, 4710-494 BRAGA (PT); RIBEIRO PIMENTA, Sara Filomena, 4835-015 GUIMARÃES (PT); FERNANDES VIEIRA, Eliana Maria, 4850-427 TABUAÇAS (PT); CARPINTEIRO ESTEVES, Bruno Miguel, 4700-752 PALMEIRA BRG (PT); MARTINS FREITAS, João Rui, 4820-139 FAFE (PT); OLIVEIRA RODRIGUES, José Artur, 4700-400 BRAGA (PT)
(74) Representative: Patentree

(57) **Abstract**

The present disclosure refers to a flexible and small-scale neuronal probe device for neuroscience studies, besides clinical monitoring and diagnosis of several neuronal pathological conditions. The mentioned device is intended for optogenetic applications and neuronal activity monitoring in an animal. More specifically, the device comprises microelectrodes for neuronal activity recording, light-emitting diodes that perform the excitation of target neurons and the excitation of a fluorophore present in those neurons, and a photodiode for reading the light emitted by the excited target neurons. The light-emitting diodes are equipped with micro-lenses for light collimation, while the photodiode is provided with a highly efficient and selective optical filter for reading, with high specificity and sensitivity, the fluorescent signal emitted by the target neurons.

## Description

### TECHNICAL FIELD

The present disclosure relates to medical devices technical field. Particularly, it relates to a flexible neuronal probe for optogenetic activation and neuronal activity monitoring in an animal, through the fabrication and integration of several optical and electrical components. The present disclosure further relates to the probe clinical use in the monitoring and diagnosis of several pathological conditions associated with the neuronal system.

### BACKGROUND

Neuronal probes are currently the most used medical devices in the study of the brain. The majority of the neuronal probes can incorporate: microelectrodes, for neuronal activity recording or stimulation, optical components, for neuronal activity stimulation or inhibition using light, or optical components, for neuronal activity reading and monitoring. The great advantage of developing a neuronal probe with electrical and optical interactions is the possibility of obtaining devices with high spatial and temporal resolutions.

Silicon (Si) is not a biocompatible material, but it is one of the most used substrates for the implementation of neuronal probes. This leads to the necessity of Si neuronal probes passivation with a biocompatible film, before their insertion into the brain tissue. More recently, there is a growing interest in using biocompatible polymers as substrates for neuronal probes microfabrication, as an alternative to Si. In addition, there are flexible polymeric based materials that can be used as neuronal probes substrates. Specifically, polymers based on polyimides, dimethyl polysiloxane (PDMS), Parylene-C and epoxy resins are the most used. The great advantage of flexible substrates, compared with rigid ones as Si, is their ability to adapt to the brain tissue micromotions and plasticity, leading to the decrease of tissue damage and improving the probe-brain interface. However, flexible polymers can increase the difficulty of probe insertion in the brain region of interest. To overcome this issue, there are already some methodologies to temporarily increase the probes rigidity, making easier their insertion.

Some of the neuronal probes with optical interaction incorporate waveguides for light delivering and collection. The documents US20120287420A1, US20150018901A1, WO2011068696A2 are examples of this type of neuronal probes. Waveguides are optical components that can be easily patterned in the neuronal probes, even with reduced dimensions. However, neuronal probes with waveguides present some disadvantages comparing with the neuronal probes with light-emitting diodes, since the neurons irradiation is not direct, reducing the light intensity that reaches the neuronal cells. This can be critical since optogenetic activation requires a minimum irradiation of 1 mW/mm². In addition, using waveguides for light delivering and collection, the light source and reading element must be outside of the probe and perfectly aligned with the waveguide, to minimize optical losses.

The document US2014/0350375A1 describes a neuronal probe implemented in a cylindrical substrate, containing several microelectrodes and, at least, one light emitting diode or photodiode. The probe diameter is equal or less than 60 µm, the microelectrodes have a diameter of 5 µm or less and the spatial resolution of the probe is, at least, 75 µm.

The document US2015/0148643A1 describes a light-emitting medical device and its manufacturing method for optogenetic applications. The medical device can be a neuronal probe integrating, at least, one GaN light-emitting diode, a detector and electrodes. This document also mentions the use of a lens or another device for collimation of the light emitted by the diode. The document US2015/0148643A1 focuses mainly on the fabrication of the light-emitting diode, without specifying the electrodes and detector type, nor their implementation. There is no specification in this document about the light detection system of the neuronal probe.

The document US2018/0193663A1 describes a neuronal probe for optogenetic stimulation and electrochemical recording, using a set of light-emitting diodes and carbon electrodes. The document focuses on the distribution and implementation of the light-emitting diodes and electrodes along the probe structure. Thus, the document does not reveal, and not even suggest, the implementation of a light detection system in the neuronal probe for neuronal activity monitoring.

The document US2019/0053712A1 describes an implantable medical device for optogenetic applications. The device contains a needle with light-emitting diodes and a photodetector or photodiode. The optical sources emit electromagnetic radiation (at 470 nm and 540 nm wavelengths) and the inorganic photodetector measures the fluorescent light emitted by the target tissue. The target tissues for this type of device are: brain, heart, kidney, eyes, skin, etc. Moreover, the document refers the presence of an optical filter in the photodetector to increase the efficiency of the collecting signal, and the use of polyimide as a substrate for the device fabrication.

Among the most recent technological advances, there is a previous study, S. Pimenta et al., "High neural selectivity probe based on a Fabry-Perot optical filter and a CMOS silicon photodiodes array at visible wavelengths", Journal of Biomedical Optics 23 (10), 2018, which describes the design, fabrication and characterization of a Fabry-Perot optical filter based on the deposition of dielectric thin-films on top of a Si photodiode matrix, integrated on a Si based neuronal probe. In-vitro fluorescence experiments showed a good performance of the set (optical filter and Si photodiode) in detecting the fluorescent signal emitted by a molecule with peak emission at ≈ 570 nm.

Despite all the studies and discussions, even today there is a growing interest in new neuronal technologies, to increase the accuracy of the recorded data and therefore improve the knowledge about the behavior of neuronal circuits.

There are several neuronal devices for neuronal activity stimulation and monitoring. However, analyzing the state of the art, there is a clear drawback related to the development of a flexible and small-scale device with simplified implementation and a light detection system with improved specificity and sensitivity. The light detection system of the present disclosure will be capable of enhancing the capacity of the neuronal probes to optically monitor neuronal activity.

### SUMMARY OF THE INVENTION

The present disclosure refers to a medical device for studies in the neuroscience field. More specifically, it refers to a neuronal device with high specificity and sensitivity in the detection of the light emitted by the neuronal tissue in an animal. The selected materials and fabrication techniques allow a simplified and fast device implementation. The present device is fabricated on a flexible and photosensitive substrate, obtaining a small-scale device for optogenetic applications and neuronal activity monitoring. The flexibility of the device substrate allows its suitability to the brain tissue micromotions, leading to the decrease of the tissue damage and improving the probe-brain interface. The substrate photosensitivity allows the patterning of the device body and its encapsulation only using photolithography, avoiding the use of complex etching techniques. This invention is related to the device clinical use for monitoring and diagnosis of several pathological conditions, such as degenerative neurological diseases, headache, tremors, depression, etc.

The present disclosure relates to a neuronal device implemented on a planar substrate, making easier the patterning of the electrical and optical components through microfabrication processes. Particularly, also relates to the fabrication of a photodiode and optical filter on its top, both based on the thin-films deposition.

The present disclosure, also relates to the use of a specific type of polyimide, the photosensitive polyimide, as substrate for the device. Photosensitive polyimide makes easier the patterning of the device body and its encapsulation, avoiding the use of complex etching techniques, commonly used with conventional (non-photosensitive) polyimide.

In the present disclosure, the implementation of a photodiode that could be fabricated only considering different thicknesses and annealing conditions of a metallic thin-film. The implementation of the photodiode based on thin-films deposition, avoids the use of semiconductor doping techniques for the implementation of Si based photodiodes.

It also relates to a highly selective optical filter (based on a Fabry-Perot structure), to increase the specificity and sensitivity of the light detection system of the device.

The present disclosure relates to a neuronal probe device for optogenetic and neuronal activity monitoring in an animal, comprising:
a flexible and photosensitive substrate;
a photodiode for reading light emitted by excited target neurons;
wherein the photodiode comprises a deposited n-type SnOₓ thin-film and a deposited p-type SnOₓthin-film.

In an embodiment, the photodiode further comprises a deposited amorphous Si (a-Si) thin-film in-between said SnOₓ thin-films.

In an embodiment, the photodiode is obtainable by thin-film deposition by thermal evaporation followed by annealing of the SnOₓthin-film to form the n-type SnOₓ thin-film with a thickness between 100 nm and 300 nm, preferably annealing at a temperature between 330 °C and 350 °C, for 2h, at a heating/cooling rate of 5 °C/min.

In an embodiment, the photodiode is obtainable by thin-film deposition by thermal evaporation followed by annealing of the SnOₓthin-film to form the p-type SnOₓ thin-film with a thickness between 500 nm and 700 nm, preferably annealing at a temperature between 225 °C and 250 °C, for 3h, at a heating/cooling rate of 5 °C/min.

In an embodiment, the photodiode is for capturing 570 nm wavelength light.

In an embodiment, the substrate is photosensitive polyimide.

In an embodiment, the substrate is planar.

In an embodiment, de device further comprises a Fabry-Perot thin-film multilayer optical filter arranged on the photodiode for filtering the light emitted by the excited target neurons, the filter comprising alternating higher TiO₂ and lower SiO₂ refractive-index deposited thin-films layers including a central resonance layer with a thickness predetermined based on filter light-wavelength to be transmitted.

In an embodiment, the device further comprises a first light-emitting diode for photoexcitation of the target neurons and a second light-emitting diode for excitation of a fluorophore present in the target neurons, in particular the first light-emitting diode having 470 nm wavelength and the second light-emitting diode has 540 nm wavelength.

In an embodiment, the neuronal device further comprises a polymeric micro-lens on a light-emitting diode for collimating light, wherein the micro-lens is a micro-lens moulded with a polymeric micro-lens mould of a same polymer as the micro-lens, in particular the polymer being dimethylpolysiloxane, PDMS.

In an embodiment, the polymeric micro-lens mould is a photosensitive polymeric mould obtained using photolithography and thermal reflow.

In an embodiment, the neuronal device further comprises one or more microelectrodes for neuronal electrical activity recording.

In an embodiment, the neuronal device further comprises tracks and contact pads for reading and actuating optical and electrical components of the device.

In an embodiment, the microelectrodes, tracks and contact pads are patterned by the photolithography and deposition of thin-films.

It is also disclosed the use of the neuronal probe device described previously, for monitoring and diagnosis of pathological conditions selected from neurological degenerative diseases selected from Alzheimer's, Parkinson's, amyotrophic lateral sclerosis (ALS), muscular dystrophy, spinal muscularatrophy (SMA), headache, migraine, tremor, depression, dementia, among others.

For a better understanding of the disclosure, it should be noted that the neuronal device may also be referred as neuronal probe.

### BRIEF DESCRIPTION OF THE DRAWINGS

The following figures provide preferred embodiments for illustrating the disclosure and should not be seen as limiting the scope of invention.
**Figure 1** represents an embodiment of the structure of the neuronal probe integrating several optical and electrical components on a substrate **1,** particularly light-emitting diodes **2** with micro-lenses **3,** photodiode **4** with optical filter **5,** microelectrodes **6,** tracks **7** and contact pads **8.**
**Figure 2** represents a preferred embodiment of the structure of the photodiode based on a p-n junction, consisting of a n-type SnOₓ and a p-type SnOₓ thin-films.
**Figure 3** represents an embodiment of the structure of the photodiode based on a p-i-n junction, consisting of a n-type SnOₓ, a p-type SnOₓ, and an amorphous Si (a-Si) thin-film as the intermediate layer (i).
**Figure 4** represents an embodiment of the Fabry-Perot structure of the optical filter on the top of the photodiode, consisting of a thin-film multilayer of titanium dioxide (TiO₂) and silicon dioxide (SiO₂).
**Figure 5** represents an embodiment of the three main steps involved in the PDMS micro-lenses fabrication, which comprises the fabrication of: the photosensitive polymeric mould using photolithography and thermal reflow; the PDMS mould based on the first polymeric mould; and the PDMS micro-lenses based on the second mould also in PDMS.

### DETAILED DESCRIPTION

The present invention refers to a medical device for studies in the neuroscience field. More specifically, it refers to a neuronal device with high specificity and sensitivity in the detection of the light emitted by the neuronal tissue in an animal. The selected materials and fabrication techniques allow a simplified and fast device implementation, obtaining a neuronal device with high spatial and temporal resolutions.

The present device is fabricated on a flexible and photosensitive substrate, obtaining a small-scale device for optogenetic applications and neuronal activity monitoring in an animal, through the fabrication and integration of several optical and electrical components.

Generally, for the optical components, the light-emitting diodes promote the excitation of the target neurons and the excitation of a fluorophore present in those neurons, while the photodiode captures the light emitted by the excited target neurons.

In an embodiment, the neuronal device dimensions can vary between: 400-600 µm wide, 100-150 µm thick and 6-8 mm needle length.

The substrate geometry was chosen considering the best interface to obtain the target information.

In an embodiment, the neuronal device presents a planar geometry. Additionally, the substrate must be biocompatible and biostable (mechanically and chemically). Specifically, the substrate must not be able to generate any type of cytotoxic, allergic, or immune reaction to the animal, should not be harmful to enzymes, cells, or tissues, must not contain mutagenic agents, neuronal tissue should not generate encapsulation nor rejection, and should not be susceptible to biological fluids or metabolic substances.

In an embodiment, the polymer used as a substrate of the neuronal device is the photosensitive polyimide.

In another embodiment the photosensitive polyimide can be replaced with any other polymer that has known adaptability to the microfabrication processes; that presents a degree of mechanical flexibility compatible with the animal movements; and that presents high biocompatibility.

In an embodiment, the light-emitting diodes have emission peaks at approximately 470 nm wavelength for target neurons photostimulation, and at approximately 540 nm wavelength for excitation of a fluorophore present in those neurons. If the target neurons are photostimulated, the fluorescent signal emitted by the fluorophore will have an emission peak at approximately 570 nm wavelength. The emitted fluorescent signal will be filtered and detected by the optical filter and photodiode, respectively.

Particularly, the light-emitting diodes are integrated into the neuronal device by permanent fixation techniques, such as welding with solder paste. The top surface of each light-emitting diode is equipped with at least one micro-lens, which is positioned for light collimation. The light collimation can improve irradiation of target neurons without increasing the energy consumption of the light-emitting diodes, i.e., it can ensure the irradiation of 1 mW/mm² for the optogenetic activation, and at the same time minimize the temperature increase (which is a critical factor for an implantable medical device).

The micro-lenses are fabricated by standard microfabrication techniques, such as photolithography, thermal reflow, and polymers demoulding.

In an embodiment, the micro-lenses material is a polymer with high transmittance in the visible region of the electromagnetic spectrum.

The emitted fluorescent signal is collected with a photodiode, which is fabricated by thin-films deposition and annealing processes, forming a p-n junction or a p-i-n junction. The used materials are tin (Sn) and a-Si.

The structure of the photodiode based on the p-n junction starts with the deposition, by thermal evaporation, of a Sn thin-film, followed by the annealing of the metallic thin-film to form the n-type SnOₓ, with a thickness between 100 nm and 300 nm, approximately. Preferably, the annealing process is performed at a temperature between 330 °C and 350 °C, for 2h, at a heating/cooling rate of 5 °C/min.

Then, a second Sn thin-film is deposited, again by thermal evaporation, on the top of the n-type SnOₓ thin-film. The resulting structure is subsequently annealed at a temperature between 225 °C and 250 °C, for 3h, at a heating/cooling rate of 5 °C/min, allowing the formation of a p-type SnOₓ thin-film with a thickness between 500 nm and 700 nm, approximately.

In an embodiment, the photodiode structure based on a p-n junction comprises at least two thin-films, a n-type SnOₓ thin-film and a p-type SnOₓ thin-film. However, the structure of the photodiode based on a p-i-n junction, in addition to the n-type SnOₓ and p-type SnOₓ, also includes the deposition of an intermediate thin-film of a-Si, for the formation of the i layer of the p-i-n junction.

The p-i-n junction starts with the deposition, also by thermal evaporation, of a Sn thin-film and subsequent annealing of the metallic thin-film at a temperature between 330 °C and 350 °C, for 2h, at a heating/cooling rate of 5 °C/min, forming the n-type SnOₓ, with a thickness between 300 nm and 500 nm, approximately.

An expert will recognize that any substantial variation in the annealing process conditions described above will significantly affect the device efficiency but these process conditions can be straightforwardly adjusted by the skilled person using common general knowledge in the field. For example, different annealing times and temperatures can be adjusted, taking into consideration that time and temperature are inversely proportional.

Then, an a-Si thin-film with a thickness between 400 nm and 600 nm, approximately, is deposited on the top of the n-type SnOₓ, using radiofrequency (RF) sputtering, forming the i layer of the p-i-n junction.

Finally, a Sn thin-film is deposited, again by thermal evaporation on the top of the a-Si thin-film. The resultant structure is subsequently annealed at a temperature between 225 °C and 250 °C, for 3h, at a heating/cooling rate of 5 °C/min, allowing the formation of a p-type SnOₓ thin-film, with a thickness between 500 nm and 700 nm, approximately.

The photodiode is equipped, on its top, with a highly selective optical filter that allows to discard the light emitted from the light-emitting diodes, and reading only the fluorescent signal emitted by neuronal tissue. The target neurons respond to the photostimulation, being possible to check its activation by measuring the intensity of the fluorescent signal emitted by the tissue at 570 nm wavelength, approximately.

The optical filter is based on a Fabry-Perot structure, implemented through the deposition, by RF sputtering, of a thin-film multilayer, specifically dielectric thin-films of TiO₂ and SiO₂. In particular, the optical filter is fabricated by alternating the deposition of high (TiO₂) and low (SiO₂) refractive index thin-films. An expert recognizes that the number of layers of the optical filter is defined according to a compromise between several features: filter transmittance, filter selectivity and fabrication process complexity.

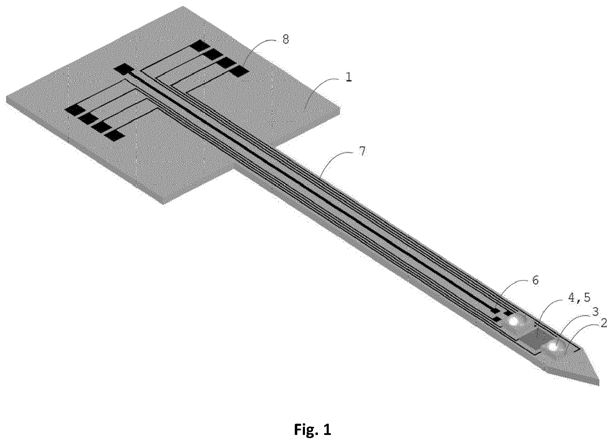

The patterning of the microelectrodes, tracks and contact pads is performed by photolithography and metallic thin-films deposition. More specifically, **Figure 1** shows a preferred embodiment of the device in which the neuronal device comprises: a substrate **1;** light-emitting diodes **2;** micro-lenses **3;** photodiode **4;** optical filter **5;** microelectrodes **6;** tracks **7;** and contact pads **8.**

In an embodiment, the device substrate **1** is a photosensitive and flexible polyimide structure, with a planar geometry, in which all the optical and electrical components of the device are patterned using photolithography and/or integrated into the substrate.

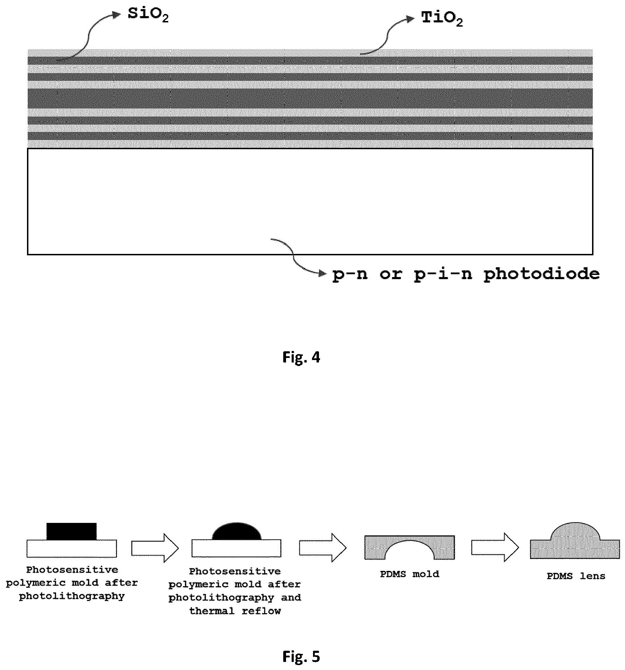

At the substrate **1,** there are at least two light-emitting diodes **2** equipped with micro-lenses **3,** positioned on the top of each light-emitting diode. In a preferred embodiment, the micro-lenses **3** are fabricated in dimethylpolysiloxane (PDMS), using photolithography and thermal reflow of a thick and chemically amplified photosensitive positive polymer, and PDMS demoulding as shown in **Figure 5****.**

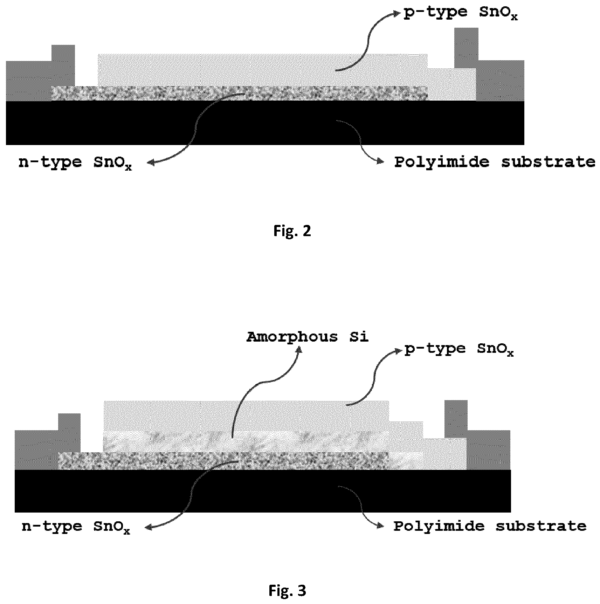

In an embodiment, the photodiode structure **4** based on a p-n junction, as shown in **Figure 2****,** is formed by the deposition of at least two metallic thin-films of Sn, using thermal evaporation. The annealing of the first Sn thin-film to form the n-type SnOₓ occurs at a temperature of approximately 330 °C, for 2h, at a heating/cooling rate of 5 °C/min. The annealing of the final structure to form the p-type SnOₓ thin-film, occurs at a temperature of approximately 250 °C, for 3h, at a heating/cooling rate of 5 °C/min. In one preferred embodiment, the n-type SnOₓ thin-film has a thickness of approximately 150 nm and the p-type SnOₓ thin-film a thickness of approximately 500 nm.

In an embodiment, the photodiode structure **4** based on a p-i-n junction as shown in **Figure 3****,** in addition to the deposition of two Sn thin-films, by thermal evaporation, also comprises the deposition of an a-Si thin-film, by RF sputtering, for the formation of the i layer of the p-i-n junction. In a preferred embodiment, this structure begins with the deposition of a Sn thin-film and subsequent annealing of the metallic thin-film at a temperature of approximately 330 °C, during 2h, at a heating/cooling rate of 5 °C/min, forming a n-type SnOₓ thin-film, with a thickness of approximately 400 nm. Subsequently, an a-Si thin-film is deposited with a thickness of approximately 400 nm, forming the i layer of the p-i-n junction. Finally, another Sn thin-film is deposited. The resulting structure is subsequently annealed at a temperature of approximately 250 °C, during 3h, at a heating/cooling rate of 5 °C/min, allowing the formation of a p-type SnOₓ thin-film, with a thickness of approximately 500 nm.

The optical filter **5** based on a Fabry-Perot structure, as shown in **Figure 4** comprises an alternating thin-film multilayer of dielectric thin-films of TiO₂ and SiO₂, which are arranged so that the central layer is the resonance layer (with a thickness oriented to the filter transmitted wavelength), and the remaining layers on each side have symmetry in terms of thicknesses. In one preferred embodiment, the multilayer should contain, at least, eleven thin-films, being the final number of layers defined according to a compromise between several features: filter transmittance, filter selectivity, and fabrication process complexity.

The optical filter **5** gives to the photodiode **4** a high selectivity and sensitivity to the fluorescent light emitted by the tissue at 570 nm wavelength, approximately, discarding the light from the light-emitting diodes. The deposition of the dielectric thin-films of the optical filter is performed by RF sputtering.

The microelectrodes **6** are used for neuronal electrical activity recording. The tracks **7** and the contact pads **8** are used to read or actuate all the optical and electrical components of the device. In a preferred embodiment, the microelectrodes **6,** tracks **7** and contact pads **8** are patterned using photolithography, with a negative photosensitive polymer based on Novolac, and metallic thin-films deposition of titanium (Ti), aluminum (Al) and platinum (Pt). The Al and Ti thin-films are deposited by electron beam (e-beam) and the Pt thin-films by direct current (DC) sputtering. This metallic multilayer structure ensures good conductivity for light-emitting diodes actuation and neuronal signals recording.

In a preferred embodiment, the neuronal device has the following dimensions, approximately: 400 µm wide, 100 µm thick and 8 mm needle length. With minimized width and thickness, the brain tissue damage after insertion of the probe is very low, considering that it is also a flexible device. With a needle length maximized, it is possible to study even the deepest regions of the brain under analysis.

Another aspect of the present disclosure relates to the use of the device in the monitoring and diagnosis of several pathological conditions, such as: degenerative neurological diseases such as Alzheimer's, Parkinson's, multiple sclerosis, amyotrophic lateral sclerosis (ALS), muscular dystrophy, spinal muscular atrophy (SMA), headache, migraine, tremor, depression, dementia, among others.

In an embodiment, a neuronal device for optogenetic applications and neuronal activity monitoring in an animal, presenting the dimensions of, approximately, 400-600 µm width, 100-150 µm thickness, and 6-8 mm needle length comprises:
a. a flexible substrate **1,** biocompatible and biostable;
b. light-emitting diodes **2;**
c. micro-lenses **3;**
d. photodiode **4;**
e. optical filter **5;**
f. microelectrodes **6;**
g. tracks **7;** and
h. contact pads **8.**

In an embodiment, the neuronal device comprises a planar substrate **1** geometry, implemented with a polymer.

In an embodiment, the neuronal device uses a photosensitive polyimide as substrate.

In an embodiment, the neuronal device has, at least, two commercial light-emitting diodes **2,** one with an emission peak around 470 nm for photostimulation of target neurons, and another one with an emission peak around 540 nm for excitation of a fluorophore, and by having at least one micro-lens **3** of dimethylpolysiloxane (PDMS) on the top surface of each light-emitting diode.

In an embodiment, the neuronal device has a photodiode **4** for capturing the fluorescent light emitted by the neuronal tissue at 570 nm wavelength, fabricated by thin-films deposition, which can form a p-n junction or a p-i-n junction based photodiode.

In an embodiment, the neuronal device uses the tin (Sn) and amorphous silicon (a-Si) thin-films, deposited by thermal evaporation and radiofrequency (RF) sputtering, respectively.

In an embodiment, the p-n junction is composed by the deposition of a Sn thin-film, followed by the thin-film annealing at a temperature between 330°C-350°C, for 2h, at a heating/cooling rate of 5°C/min.

In an embodiment, it is obtained after annealing, a n-type SnOₓ thin-film with a thickness between 100 nm and 300 nm, approximately.

In an embodiment, the p-n junction comprise the deposition of a second Sn thin-film on the top of the n-type SnOₓ thin-film, followed by the annealing of the resulting structure at a temperature between 225°C and 250°C, for 3 hours, at a heating/cooling rate of 5°C/min.

In an embodiment, it is obtained after annealing, a p-type SnOₓ thin-film with a thickness between 500 nm and 700 nm, approximately.

In an embodiment, the p-i-n junction is composed by the deposition of a Sn thin-film, followed by the thin-film annealing at a temperature between 330°C and 350°C, for 2h, at a heating/cooling rate of 5°C/min.

In an embodiment, it is obtained after annealing, a n-type SnOₓ thin-film with a thickness between 300 nm and 500 nm, approximately.

In an embodiment, the p-i-n junction comprises the deposition of an intermediate film of amorphous silicon (a-Si), on the top of the n-type SnOₓ thin-film, with a thickness between 400 nm and 600 nm, approximately, and the deposition of a second Sn thin-film, on the top of the a-Si thin-film, and subsequent annealing of the structure at a temperature between 225°C and 250°C, for 3h, at a heating/cooling rate of 5°C/min.

In an embodiment it is obtained after annealing, a p-type SnOₓ thin-film with a thickness between 500 nm and 700 nm, approximately.

In an embodiment, the photodiode **4** comprises, in its top surface, a Fabry-Perot optical filter **5** to discard the light from the light-emitting diodes, and read only the fluorescent signal emitted by the neuronal tissue.

In an embodiment, the microelectrodes **6,** tracks **7,** and contact pads **8** are patterned with photolithography, with a negative photosensitive polymer based on Novolac, and thin-films deposition of titanium (Ti) and aluminum (Al), deposited by electron beam (e-beam), and platinum (Pt), deposited by direct current (DC) sputtering.

In an embodiment the neuronal device is used for monitoring and diagnosis of several pathological conditions, such as: neurological degenerative diseases such as Alzheimer's, Parkinson's, amyotrophic lateral sclerosis (ALS), muscular dystrophy, spinal muscular atrophy (SMA), headache, migraine, tremor, depression, dementia, etc.

The disclosure should not be seen in any way restricted to the embodiments described and a person with ordinary skill in the art will foresee many possibilities to modifications thereof. The above described embodiments are combinable.

The following claims further set out particular embodiments of the disclosure.

## Claims

1. Neuronal probe device for optogenetic and neuronal activity monitoring in an animal, comprising:
a flexible and photosensitive substrate;
a photodiode for reading light emitted by excited target neurons;
wherein the photodiode comprises a deposited n-type SnOₓ thin-film and a deposited p-type SnOₓ thin-film.

2. Neuronal device according to the previous claim wherein the photodiode further comprises a deposited amorphous Si (a-Si) thin-film in-between said SnOₓ thin-films.

3. Neuronal device according to any of the previous claims wherein the photodiode is obtainable by thin-film deposition by thermal evaporation followed by annealing of the SnOₓ thin-film to form the n-type SnOₓ thin-film with a thickness between 100 nm and 300 nm, preferably annealing at a temperature between 330 °C and 350 °C, for 2h, at a heating/cooling rate of 5 °C/min.

4. Neuronal device according to any of the previous claims wherein the photodiode is obtainable by thin-film deposition by thermal evaporation followed by annealing of the SnOₓ thin-film to form the p-type SnOₓ thin-film with a thickness between 500 nm and 700 nm, preferably annealing at a temperature between 225 °C and 250 °C, for 3h, at a heating/cooling rate of 5 °C/min.

5. Neuronal device according to any of the previous claims wherein the photodiode is for capturing 570 nm wavelength light.

6. Neuronal device according to any of the previous claims wherein the substrate is photosensitive polyimide.

7. Neuronal device according to the previous claim, wherein the substrate is planar.

8. Neuronal device according to any of the previous claims further comprising an Fabry-Perot thin film multilayer optical filter arranged on the photodiode for filtering the light emitted by the excited target neurons, the filter comprising alternating higher TiO₂ and lower SiO₂ refractive-index deposited thin-films layers including a central resonance layer with a thickness predetermined based on filter light-wavelength to be transmitted.

9. Neuronal device according to any of the previous claims further comprising a first light-emitting diode for photoexcitation of the target neurons and a second light-emitting diode for excitation of a fluorophore present in the target neurons, in particular the first light-emitting diode having 470 nm wavelength and the second light-emitting diode has 540 nm wavelength.

10. Neuronal device according to any of the previous claims further comprising a polymeric micro-lens on a light-emitting diode for collimating light, wherein the micro-lens is a micro-lens moulded with a polymeric micro-lens mould of a same polymer as the micro-lens, in particular the polymer being dimethylpolysiloxane, PDMS.

11. Neuronal device according to the previous claim wherein the polymeric micro-lens mould is a photosensitive polymeric mould obtained using photolithography and thermal reflow.

12. Neuronal device according to any of the previous claims further comprising one or more microelectrodes for neuronal electrical activity recording.

13. Neuronal device according to any of the previous claims further comprising tracks and contact pads for reading and actuating optical and electrical components of the device.

14. Neuronal device according to claims 12 and 13 wherein the microelectrodes, tracks and contact pads are patterned by the photolithography and deposition of thin-films.

15. Use of the neuronal probe device according to claims 1 to 14, for monitoring and diagnosis of pathological conditions selected from neurological degenerative diseases selected from Alzheimer's, Parkinson's, amyotrophic lateral sclerosis (ALS), muscular dystrophy, spinal muscular atrophy (SMA), headache, migraine, tremor, depression, dementia, among others.
